# EUROPEAN PATENT APPLICATION

(11) **EP 3 995 150 A1**
(43) Date of publication of application: **11.05.2022**
(21) Application number: 20835395.3
(22) Date of filing: 01.07.2020
(51) Int. Cl.: A61K 45/00, A61K 48/00, A61P 7/02, A61P 13/12, A61P 35/00, C07K 14/00, A61K 47/42, A61K 47/62, A61K 47/64, A61K 49/00, A61K 51/00, A61K 51/08, A61K 31/7088, A61K 31/7105, A61K 31/711, A61K 31/713

(54) **RENAL TARGETING-TYPE DRUG DELIVERY CARRIER HAVING EXCELLENT BIODEGRABILITY**

(30) Priority: 02.07.2019 JP 2019123540
(71) Applicant: Kyoto Pharmaceutical University, Kyoto-shi, Kyoto 607-8414 (JP)
(72) Inventor: KATSUMI, Hidemasa, Kyoto-shi, Kyoto 607-8414 (JP); YAMAMOTO, Akira, Kyoto-shi, Kyoto 607-8414 (JP)
(74) Representative: Heubeck, Christian
(86) International application number: PCT/JP2020/026618
(87) International publication number: WO 2021/002481

(57) **Abstract**

The present invention aims to provide a carrier for drug delivery that selectively accumulates in the kidney in the body, and shows high biodegradability and drug releasability in the kidney. The present invention relates to a compound in which a carbonyl group of serine is linked directly or via a linker to terminal amino group of linear polylysine, compound carrier for drug delivery, and a medicament for the diagnosis, prophylaxis, or treatment of a renal disease, containing the carrier for drug delivery, and a drug bound to the carrier directly or via a linker or encapsulated therein.

## Description

### [Technical Field]

The present invention relates to a carrier for kidney targeting drug delivery that selectively accumulates in kidney, particularly, proximal renal tubule, in the body, and is superior in drug releasability and biodegradability.

### [Background Art]

The development of a dosage form for most effectively and safely administering a drug by controlling the pharmacokinetics of the drug, that is, a drug delivery system, has attracted attention in recent years in drug development. However, carriers for drug delivery that selectively accumulate in the kidney have hardly been developed.

For example, drugs modified with succinic acid or aconitic acid are known to accumulate relatively easily in the kidney, but they also accumulate in the liver at the same time (non-patent document 1).

In addition, polyvinylpyrrolidone-type compounds have drawn attention as targeting elements that selectively accumulate in the kidney. However, since they are artificial polymers, decomposition thereof is difficult after being distributed to the kidney, thus raising a concern about accumulation properties and safety. Therefore, clinical application thereof as kidney targeting elements is difficult and has not been put to practical use (non-patent document 2).

Renal disease includes various diseases such as glomerulonephritis, IgA nephropathy, diabetic nephropathy, membranous nephropathy, hydronephrosis, contrast nephropathy, pyelonephritis, renal failure, acute nephritis, chronic nephritis, uremia, interstitial nephritis, kidney disease, nephrotic syndrome, hypertensive nephrosclerosis, diabetic glomerulosclerosis, kidney calculus, amyloid nephropathy, renal vein thrombosis, Alport syndrome, renal tumor and the like. Therefore, development of a carrier for drug delivery that selectively accumulates in the kidney is extremely important in treating these diseases.

Recently, the present inventors have found that a serine-modified dendrimer in which the amino acid, serine, is bound to a dendrimer (polyamideamine dendrimer (PAMAM)) selectively accumulates in the kidney, particularly proximal renal tubule, and reported that it is promising as a kidney targeting carrier for drug delivery (patent document 1, non-patent documents 3, 4) .

However, in general, dendrimers have limited usefulness in humans due to the toxicity thereof, difficulty in synthesis, cost, and the like (non-patent document 5).

### [Document List]

### [Patent documents]

Patent document 1: WO 2019/009436

### [Non-patent documents]

Non-patent document 1: Yamasaki, Y. et al., J. Pharmacol. Exp. Ther., 2002 May; 301(2):467-477
Non-patent document 2: Kamada, H. et al., Nat Biotechnol., 21(4):399-404 (2003).
Non-patent document 3: Matsuura, S. et al., Proc. Natl. Acad. Sci. USA., 115(41):10511-10516 (2018).
Non-patent document 4: Matsuura, S. et al., Pharmaceutics, 10 (4) :pii: E251 (2018).
Non-patent document 5: Cheng, Y. et al., Chem. Soc. Rev., 40:2673-2703 (2011).

### [Summary of Invention]

### [Problems to be solved by the Invention]

Under such circumstances, in practicalization of a carrier for drug delivery, there is an increasing demand for the development of a carrier for drug delivery that is superior in safety and commercial availability, shows high kidney targeting efficiency, and is superior in biodegradability in kidney.

The present invention aims to provide a safe and practical carrier for drug delivery that has high biocompatibility, selectively accumulates in kidney, and has superior biodegradability by using a biological component, serine, as a kidney targeting element, and binding to another polymer instead of the dendrimer. The present invention further aims to provide a safe and effective diagnostic, prophylactic or therapeutic agent for renal diseases, which is composed of the carrier for drug delivery and a drug bound thereto or encapsulated therein.

### [Means of Solving the Problems]

Under these circumstances, the present inventors have conducted intensive studies and found for the first time that a compound, in which the carbonyl group of serine is linked by a peptide bond or an ester bond directly or via a linker to each of at least 50% of the total number of the terminal amino groups of linear polylysine (hereinafter sometimes to be referred to as "the compound of the present invention"), and a medicament containing the compound to which a drug is bound directly or via a linker, or a drug is encapsulated therein can accomplish highly selective renal distribution and high kidney targeting efficiency along with superior biodegradability and drug releasability in kidney (hereinafter sometimes to be referred to as "the medicament of the present invention"), which resulted in the completion of the present invention.

Therefore, the present invention provides the following.
[1] A compound in which a carbonyl group of serine is linked by a peptide bond or an ester bond directly or via a linker to at least 50% of the total number of terminal amino groups of linear polylysine.
[2] The compound of the above-mentioned [1], wherein the carbonyl group of serine is directly linked to the terminal amino group of linear polylysine by a peptide bond.
[3] The compound of the above-mentioned [1] or [2], wherein the linear polylysine has a number average molecular weight of 4000 - 15000Da.
[4] A carrier for drug delivery that is composed of the compound of any of the above-mentioned [1] to [3], and selectively delivers a drug to a target tissue in vivo.
[5] The carrier for drug delivery of the above-mentioned [4], wherein the aforementioned target tissue is kidney.
[6] A medicament comprising the carrier for drug delivery of the above-mentioned [4] or [5], and a drug bound to the carrier directly or via a linker or encapsulated therein.
[7] The medicament of the above-mentioned [6], wherein the aforementioned drug is at least one selected from the group consisting of an angiotensin converting enzyme inhibitor, an anti-cancer agent, an anti-inflammatory agent, an anti-infective agent, an anti-fibrotic agent, an immunosuppressant, an antioxidant, a nucleic acid drug, a radiopharmaceutical and a contrast agent.
[8] The medicament of any of the above-mentioned [6] or [7], wherein the medicament is a diagnostic, prophylactic or therapeutic agent for renal diseases.
[9] The medicament of the above-mentioned [8], wherein the renal disease is at least one member selected from the group consisting of glomerulonephritis, IgA nephropathy, diabetic nephropathy, membranous nephropathy, hydronephrosis, contrast nephropathy, pyelonephritis, renal failure, acute nephritis, chronic nephritis, interstitial nephritis, renopathy, nephrotic syndrome, hypertensive nephrosclerosis, diabetic glomerulosclerosis, kidney calculus, amyloid nephropathy, renal vein thrombosis, Alport syndrome, and renal tumor.

### [Effect of the Invention]

The compound of the present invention can be easily synthesized using a commercially available linear polylysine as it is as a starting material. The compound of the present invention shows high renal selectivity with a particularly high renal distribution rate and scarce distribution to other organs, and are extremely useful as a carrier for drug delivery of prophylactic agents, diagnostic agents (clinical test drugs), or therapeutic agents for various renal diseases. Furthermore, the compound of the present invention and a medicament in which a drug is bound to or encapsulated in the compound is superior in the biodegradability in the kidney and releasability of the serine moiety that binds to the drug. Therefore, they are expected to be applicable to a wide range of use as a low toxic, practical carrier for drug delivery, and a safe and effective medicament.

### [Brief Description of the Drawings]

Fig. 1 shows concentration-time profiles in plasma, liver, kidney, spleen, heart and lung after intravenous injection of ¹¹¹In-labeled poly-L-lysine (compound of Control Example) (A) and ¹¹¹In-labeled serine-modified poly-L-lysine (¹¹¹In-labeled compound (Ia)) (B) to ddY mouse.
Fig. 2 shows SPECT/CT images of organ distribution 3 hr after intravenous injection of ¹¹¹In-labeled serine-modified poly-L-lysine (¹¹¹In-labeled compound (Ia)). A shows a 3D image, B shows an image of a sagittal plane, C shows an image of a coronal plane, and D shows an image of a transverse plane.
Fig. 3 shows a microscopic image of a kidney section of a mouse 60 min after intravenous administration of FITC-labeled serine-modified poly-L-lysine (FITC-labeled compound (Ia)). A shows an image of renal cortex, and B shows an enlarged image of renal cortex.
Fig. 4 shows radioactivity in high molecular weight fraction and low molecular weight fraction in kidney homogenate samples before administration (A) and 3 hr after administration (B) of ¹¹¹In-labeled serine-modified PAMAM(G3) (compound of Comparative Example 1).
Fig. 5 shows radioactivity in high molecular weight fraction and low molecular weight fraction in kidney homogenate samples before administration (A) and 3 hr after administration (B) of ¹¹¹In-labeled serine-modified poly-L-lysine (¹¹¹In-labeled compound (Ia)).
Fig. 6 shows radioactivity in high molecular weight fraction and low molecular weight fraction in kidney homogenate samples before administration (A) and 3 hr after administration (B) of ¹¹¹In-labeled PAMAM(G3) (compound of Comparative Example 2) .
Fig. 7 shows radioactivity in high molecular weight fraction and low molecular weight fraction in kidney homogenate samples before administration (A) and 3 hr after administration (B) of ¹¹¹In-labeled poly-L-lysine (compound of Control Example).
Fig. 8 shows plasma creatinine concentration (A), plasma urea nitrogen concentration (B), and histological section of kidney tissue of each administration group (C), after intravenous injection of serine-modified poly-L-lysine (compound (Ia)) into ddY mice for 5 consecutive days.
Fig. 9 shows a renal cell carcinoma proliferation suppressive effect compared with a PBS administration group after intravenous administration of ⁹⁰Y-labeled serine-modified poly-L-lysine (⁹⁰Y-labeled compound (Ia)) to renal cell carcinoma model mice. (decrease in the number of cancer cells (A), suppression of cancer cell-derived luciferase luminescence (B), and change in the body weight of mouse (C)).

### [Description of Embodiments]

The detail of the present invention is described in the following.

### (Definition)

In the present specification, the "linear polylysine" means a linear ε-poly-L-lysine represented by the following formula:

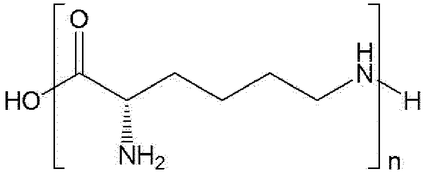

wherein n is not less than 10 (sometimes to be simply referred to as "poly-L-lysine" in the present specification), and is a homopolypeptide of L-lysine residue.

The number average molecular weight of such linear polylysine is not particularly limited as long as it does not prevent administration to a living body. It is about 1000 - 50000 Da, preferably about 3000 - 30000 Da, more preferably about 4000 - 15000 Da. Linear polylysines in various molecular weight ranges can be obtained as commercially available products.

In the present specification, "at least 50% of the total number of terminal amino groups" means a number of at least 50% (not less than 50%) of the total number of the terminal amino groups of the aforementioned linear polylysine.

In the present specification, the "terminal amino group" includes not only an amino group of the long chain terminal of linear polylysine but also α-amino group existing as many as the number of lysines constituting polylysine.

In the present specification, the "linker" means a bifunctional (homobifunctional or heterobifunctional) or polyfunctional chemical moiety containing a chain of atoms that covalently (by peptide bond or ester bond) connects the carboxy group of serine to the terminal amino group of a linear polylysine (hereinafter sometimes to be referred to as "the first linker"), or a chain of atoms, inclusion site or chelating site that covalently or noncovalently links a drug to a serine-derived terminal amino group from among the terminal groups of the serine-modified poly-L-lysine, or the terminal amino group of the non-serine-modified moiety of the serine-modified poly-L-lysine (hereinafter sometimes to be referred to as "the second linker").

The bifunctional linker is preferably formed from a linker reagent having the formula:

X-R-Y

wherein X is a first reactive moiety, R is a spacer, Y is a second reactive moiety. X and Y may be suitable reactive moieties which may be the same (that is, homobifunctional linker) or different (that is, heterobifunctional linker). The suitable reactive moiety includes, but is not limited to, aldehyde (formyl group), amino group, halogen, hydroxy group, carboxy group, diene, hydrazide, maleimide, NHS ester, phosphoryl group, sulfhydryl group or other reactive moiety. Preferably, either X or Y is halogen, a formyl group, a carboxy group, hydrazide, maleimide or a sulfhydryl group. The spacer (R) may be a suitable unsubstituted or substituted aliphatic or aromatic organic moiety. The suitable organic moiety may be a divalent group selected from the group consisting of carbonyl, alkyl, cycloalkyl, cycloalkylalkyl, arylalkyl, aryl, heteroaryl, alkoxyalkyl, haloaryl, hydroxyalkyl, carboxy, carboxyalkyl, alkanoyl, alkenyl and alkynyl, but is not limited thereto. The spacer preferably contains 1 - 20 carbon atoms, more preferably 2 - 12 carbon atoms.

In the present invention, the first linker or the second linker that covalently links the carbonyl group of serine or a drug to the terminal amino group may be composed of one or more bifunctional linkers. As a linker reagent that forms such bifunctional linker, linker reagents commercially available from various reagent suppliers (see, for example, catalogs of reagent suppliers such as Pierce Inc., Sigma-Aldrich Co. LLC. and the like) can be mentioned. Using these reagents and according to a method known per se, the linkers can be easily introduced by those of ordinary skill in the art. Examples of the bifunctional linker include, but are not limited to, a linker, which is formed by one or more commercially available linker reagents selected from 6-maleimidocaproyl (MC), maleimidopropanoyl (MP), p-aminobenzyloxycarbonyl (PAB), ethyleneoxy(-CH₂CH₂O-; EO or PEG) as one or more repeating units, N-succinimidyl-4-(2-pyridylthio)propanoate (SPDP), PEGylated long chain SPDP crosslinker, N-succinimidyl-[(N-maleimidopropionamide)-n ethyleneglycol]ester (SM(PEG)ₙ) (wherein n is 2, 4, 6, 8, 12, 24 etc.), N-succinimidyl-4-(N-maleimidomethyl)cyclohexane-1-carboxylate (SMCC), N-succinimidyl 4-(N-maleimidomethyl)cyclohexane-1-carboxy-6-amidocaproate (LC-SMCC), 2-iminothiolane (Traut's reagent), sulfosuccinimidyl 6-(3'-(2-pyridylthio)propionamide)hexanoate (sulfo-LC-SPDP), N-succinimidyl-4-(2-pyridylthio)pentanoate (SPP), N-succinimidyl-(4-iodoacetyl)aminobenzoic acid ester (SIAB), bis-maleimido-trioxyethylene glycol (BMPEO), N-β-maleimidopropyl-oxysuccinimide ester (BMPS), N-ε-maleimidocaproyl-oxysuccinimide ester (EMCS), N-γ-maleimidobutyryl-oxysuccinimide ester (GMBS), 1,6-hexane-bis-vinylsulfone (HBVS), m-maleimidobenzoyl-N-hydroxysuccinimide ester (MBS), 4-(4-N-maleimidophenyl)butyric acid hydrazide (MPBH), succinimidyl 3-(bromoacetamido)propionate (SBAP), succinimidyl iodoacetate (SIA), succinimidyl 4-(p-maleimidophenyl)butyrate (SMPB), succinimidyl 6-((β-maleimidopropionamido)hexanoate) (SMPH), N-ε-maleimidocaproyl-oxysuccinimide ester (sulfo-EMCS), N-γ-maleimidobutyryl-oxysuccinimide ester (sulfo-GMBS), N-[κ-maleimidoundecanoyloxy]-sulfosuccinimide ester (sulfo-KMUS), m-maleimidobenzoyl-N-hydroxysulfosuccinimide ester (sulfo-MBS), sulfosuccinimidyl (4-iodoacetyl)aminobenzoate (sulfo-SIAB), sulfosuccinimidyl 4-(N-maleimidomethyl)cyclohexane-1-carboxylate (sulfo-SMCC), sulfosuccinimidyl 4-(N-maleimidophenyl)butyrate (sulfo-SMPB), succinimidyl-(4-vinylsulfone)benzoate (SVSB), dithiobismaleimidoethane (DTME), 1,4-bismaleimidobutane (BMB), 1,4-bis-maleimidyl-2,3-dihydroxybutane (BMDB), bis(maleimido)hexane (BMH), bis(maleimido)ethane (BMOE), 1,8-bismaleimide-diethylene glycol (BM(PEG)₂), 1,11-bismaleimide-triethylene glycol (BM(PEG)₃), diethylenetriamine-N,N,N',N",N"-pentaacetic acid dianhydride (DTPA anhydride), dibenzocyclooctyne-N-hydroxysuccinimidyl ester (DBCO-NHS), dibenzocyclooctyne-n(ethyleneglycol)-N-hydroxysuccinimidyl ester (DBCO-(PEG)ₙ-NHS) (wherein n is 4, 5, 13 etc.), azido-n(ethyleneglycol)-N-succinimidyl ester (azido-(PEG)ₙ-NHS) (wherein n is 1, 2, 3, 4, 5, 6, 8, 12, 16, 24 etc.), and the like. Among these, a linker, which is formed by one or more linker reagents selected from the group consisting of N-succinimidyl-4-(2-pyridylthio)propanoate (SPDP), PEGylated long chain SPDP crosslinker, N-succinimidyl-[(N-maleimidopropionamide)-n(ethyleneglycol)]ester (SM(PEG)ₙ) (wherein n is 2, 4, 6, 8, 12, 24 etc.), N-succinimidyl 4-(N-maleimidomethyl)cyclohexane-1-carboxy-6-amidocaproate (LC-SMCC), 2-iminothiolane (Traut's reagent) and sulfosuccinimidyl 6-(3'-(2-pyridylthio)propionamide)hexanoate (sulfo-LC-SPDP) is particularly preferable.

In the present invention, examples of the second linker that retains a drug by noncovalently including in or chelating to the terminal group include a linker having a molecular recognition moiety (inclusion moiety or chelate moiety) that can be bonded to a serine-derived terminal amino group of a serine-modified poly-L-lysine, or a terminal amino group of a non-serine-modified moiety of a serine-modified poly-L-lysine, and can encapsulate a drug in the terminal. In such linker, an inclusion moiety or chelate moiety capable of encapsulating a drug is bonded to a serine-modified or non-serine-modified terminal amino group directly or via the aforementioned one or more bifunctional linkers. Such inclusion moiety or chelate moiety is not linked to a drug by a covalent bond, however, it is defined as one of the bifunctional linkers in the present specification since it can be linked to a drug by a noncovalent interaction. Examples of such inclusion moiety or chelate moiety include, but are not limited to, inclusion moieties such as 3A-amino-3A-deoxy-(2AS,3AS)-γ-cyclodextrin hydrate, cholesterol, cholic acid, C₆₀ fullerene and the like, chelate moieties such as diethylenetriamine-N,N,N',N",N"-pentaacetic acid (DTPA) and the like, and the like.

In the present specification, "the carbonyl group of serine is linked by a peptide bond or an ester bond directly or via a linker" to a terminal amino group of a linear polylysine means a state where the carboxy group of serine is linked by a peptide bond or an ester bond and introduced to each of the terminal amino groups present in plurality in the linear polylysine, or a new terminal group formed by the binding of the aforementioned first linker to each terminal amino group of the linear polylysine (amino group or hydroxy group). As the compound of the present invention and the medicament of the present invention, those in which the carboxy group of serine is directly linked to the terminal amino group of linear polylysine by a peptide bond are preferable.

In the present specification, "serine-modified" and "non-serine-modified" (or "unmodified") respectively mean a state where a terminal amino group of a linear polylysine and an α-carboxy group of serine are linked by a peptide bond or an ester bond directly or via a linker, and a state where serine is not linked to a terminal amino group of a linear polylysine or a terminal group formed by the binding of a linker to the linear polylysine.

In the compound of the present invention, or a medicament in which a drug is bound directly or via a linker to or encapsulated in the compound of the present invention (the medicament of the present invention), a serine-modified site (hydroxy group and amino group derived from serine) of the terminal amino group (or terminal group via a linker) of linear polylysine functions as a kidney targeting element. The "kidney targeting element" means a site having a biological recognition function and capable of forming a biological binding pair with the compound or medicament of the present invention by specifically binding to the kidney.

The introduction rate of serine (serine modification rate) into a terminal amino group of a linear polylysine (or a terminal group via a linker) that is required by the compound of the present invention or the medicament of the present invention to express high renal distribution and high renal selectivity is at least 50%, preferably not less than 60%, more preferably not less than 70%, particularly preferably not less than 80%, based on the total number of terminal amino groups. The introduction rate of serine can be calculated from the measurement results of proton nuclear magnetic resonance (¹H-NMR), matrix-assisted laser desorption/ionization time-of-flight mass spectrometer (MALDI-TOF-MS) or the like.

In the present specification, the "medicament" means a drug given to diagnose, prevent or treat a disease in mammals, including humans.

In the present specification, examples of the "drug" include angiotensin converting enzyme (ACE) inhibitor, anti-cancer agent, anti-inflammatory agent, anti-infective agent, anti-fibrotic agent, immunosuppressant, antioxidant, nucleic acid drug, radiopharmaceutical, contrast agent and the like. The kind of the drug is not particularly limited. Specific examples of the drug are shown in the following, though the drug is not limited to the specific examples below.

Examples of the angiotensin converting enzyme (ACE) inhibitor include captopril, imidapril, enalapril, lisinopril, benazepril, perindopril, delapril, trandolapril, cilazapril and the like.

Examples of the anti-cancer agent include BCG, actinomycin D, asparaginase, aceglatone, anastrozole, allopurinol, anthracycline, bicalutamide, anti-androgen, idarubicin, ifosfamide, imatinib, irinotecan, interferon, interferon alpha, interleukin-2, ubenimex, exemestane, estramustine, estrogen, etoposide, enocitabine, epirubicin, oxaliplatin, octreotide, capecitabine, carboquone, carboplatin, carmofur, cladribine, clarithromycin, krestin, ketoconazole, gefitinib, gemcitabine, gemtuzumab, goserelin, cyclophosphamide, cisplatin, schizophyllan, cytarabine, cyproheptadine, zinostatin stimalamer, cetuximab, sobuzoxane, tamoxifen, daunorubicin, dacarbazine, dactinomycin, thiotepa, tegafur, tegafur-uracil, tegafur-gimeracil-oteracil potassium, dexamethasone, topotecan, trastuzumab, triptorelin, tretinoin, toremifene, doxifluridine, doxorubicin, docetaxel, nimustine, neocarzinostatin, nedaplatin, paclitaxel, hydroxyurea, hydroxycarbamide, bicalutamide, vinorelbine, vincristine, vindesine, vinblastine, picibanil, pirarubicin, fadrozole, fluorouracil, flutamide, fludarabine, busulfan, bleomycin, prednisone, procarbazine, progestin, peplomycin, pentostatin, porfimer sodium, mitomycin, mitoxantrone, mitotane, mesna, methotrexate, medroxyprogesterone, mercaptopurine, melphalan, ranimustine, rituximab, leuprolide, retinoic acid, lentinan, leucovorin and the like. The above-mentioned anti-cancer agents may be used alone, or two or more kinds of anti-cancer agents may also be used in combination.

Examples of the anti-inflammatory agent include steroidal anti-inflammatory agent and non-steroidal anti-inflammatory agent.

Examples of the steroidal anti-inflammatory agent include adrenal corticosteroidal anti-inflammatory agents, for example, dexamethasone, triamcinolone acetoide, beclometasone, hydrocortisone, methylprednisolone, predonisolone, prednisone, triamcinolone diacetate, cortisone, cortisol, paramethasone, triamcinolone, diflucortolone, difluprednate, diflorasone, flumetasone, fluocinonide, fluocinolone acetonide, alclometasone, fludrocortisone and the like, and a salt thereof. More specifically, for example, dexamethasone, triamcinolone acetonide, beclomethasone dipropionate, hydrocortisone succinate, methylprednisolone succinate, dexamethasone acetate, hydrocortisone acetate, predonisolone acetate, dexamethasone metasulfonate benzoate, triamcinolone diacetate, predonisolone butylacetate, dexamethasone phosphate, hydrocortisone phosphate, prednisolone phosphate, betamethasone phosphate, predonisolone succinate, cortisone acetate, paramethasone acetate, methylprednisolone acetate, triamcinolone, hydrocortisone, predonisolone, betamethasone, prednisolone valerate, diflucortolone valerate, dexamethasone valerate, betamethasone valerate, difluprednate acetate, diflorasone acetate, difluprednate, betamethasone dipropionate, flumetasone pivalate, fluocinonide, fluocinolone acetonide, alclometasone propionate, beclomethasone dipropionate, clobetasone butyrate, hydrocortisone butyrate, hydrocortisone butyrate propionate, fludrocortisone butyrate, dexamethasone palmitate, methylprednisolone and the like can be mentioned.

Examples of the non-steroidal anti-inflammatory agent include NSAID, COX-2 inhibitor and the like. More specifically, for example, acetylsalicylic acid, alclofenac, alminoprofen, benoxaprofen, butibufen, bucloxic acid, carprofen, celecoxib, clidanac, diclofenac, diflunisal, etodolac, fenbufen, fenoprofen, fentiazic, flufenamic acid, flufenasol, flurbiprofen, furofenac, ibufenac, ibuprofen, indomethacin, indoprofen, isoxepac, isoxicam, ketoprofen, ketorolac, meclofenamic acid, mefenamic acid, meloxicam, miroprofen, naproxen, oxaprozin, oxyphenbutazone, oxypinac, parecoxib, phenylbutazone, piclamilast, piroxicam, pirprofen, pranoprofen, rofecoxib, sudoxicam, sulindac, suprofen, tenclofenac, tiaprofenic acid, tolfenamic acid, tolmetin, tramadol, valdecoxib, zomepirac, and the like, and a salt thereof and the like can be mentioned.

Examples of the anti-infective agent include levofloxacin, ceftriaxone, minocycline, sulfamethoxazole, trimethoprim and the like.

Examples of the anti-fibrotic agent include atrial natriuretic peptide, pyridone derivative, pirfenidone, pantethine, cysteine, histidine, S-allylcysteine, growth factor, HGF (Hepatocyte growth factor), beperminogene perplasmid, osteoactivin, aldosterone antagonist, ACE inhibitor, imidapril hydrochloride, keratan sulfate oligosaccharide and the like.

Examples of the immunosuppressant include rapamycin, tacrolimus, cyclosporine, predonisolone, methylprednisolone, mycophenolate mofetil, azathioprine, mizoribine and the like.

Examples of the antioxidant include superoxide dismutase, catalase, nitric oxide donor, hydrogen sulfide donor, curcumin, coenzyme Q10, astaxanthin, α-tocopherol, α-tocopherol derivative and the like.

Examples of the nucleic acid drug include siRNA, anitisense, plasmid DNA, mRNA and the like.

Examples of the radiopharmaceutical and contrast agent include yttrium Y-90, gadolinium Ga-67, gadolinium Ga-68, lutetium Lu-177, copper Cu-64, technetium Tc-99, Rhenium Re-186, Rhenium Re-188, indium-111, fluorine-18, radioactive iodine-131 and the like.

In the present specification, examples of the "renal diseases" include glomerulonephritis, IgA nephropathy, diabetic nephropathy, membranous nephropathy, hydronephrosis, contrast agent nephropathy, pyelonephritis, renal failure, acute nephritis, chronic nephritis, uremia, interstitial nephritis, kidney disease, nephrotic syndrome, hypertensive nephrosclerosis, diabetic glomerulosclerosis, kidney calculus, amyloid kidney, kidney intravenous thrombosis, Alport syndrome, kidney tumor and the like.

In the present specification, the "treatment" means administering the medicament of the present invention to an individual (e.g., a mammal including a human) who has already developed a renal disease for the purpose of curing the disease, alleviating the disease, preventing the disease from worsening, or preventing seizures, and the "prophylaxis" means administering the medicament of the present invention to a healthy individual (e.g., a mammal including a human) who has not developed a renal disease or related symptoms for the purpose of preventing the onset of the disease.

In the present specification, the "mammals including humans" means a human or non-human mammal (e.g., monkey, bovine, horse, dog, sheep, swine, rabbit, cat, mouse, rat etc.).

In the present specification, the "drug is bound directly or via a linker" means a state where the drug is bound by a covalent bond directly or via the aforementioned one or more bifunctional linkers (the second linker) to a serine-derived amino group on the serine-modified terminal or an amino group or a hydroxy group on a non-serine-modified terminal of the compound of the present invention.

In the present specification, the "drug is encapsulated" means a state where the aforementioned second linker having a molecular recognition moiety (e.g., inclusion moiety, chelate moiety etc.) at the terminal is bonded to a serine-derived amino group on the terminal of the compound of the present invention directly or via a linker (the aforementioned second linker), and a drug is non-covalently incorporated into the molecular recognition moiety of the compound of the present invention by hydrophobic interaction or chelating bond (i.e., included or chelated state).

The loading rate or inclusion rate of the drug in the medicament of the present invention can be appropriately changed depending on the kinds of the drug and linker. It is generally about 1 - 40%, preferably about 5 - 20%, of the total number of the terminal groups of the compound of the present invention. The loading rate or inclusion rate of the drug can be calculated from the measurement results of proton nuclear magnetic resonance (¹H-NMR), matrix-assisted laser desorption/ionization time-of-flight mass spectrometer (MALDI-TOF-MS), trinitrobenzenesulfonic acid (TNBS) method (Habeeb AF. Anal. Biochem., 14, 328-336 (1966)), or the like.

The compound of the present invention preferably includes the following compounds.

### [Compound (A)]

The compound of the present invention, wherein the carbonyl group of serine is linked by a peptide bond or ester bond, directly or via a linker, to at least 50% (preferably not less than 60%, more preferably not less than 70%, particularly preferably not less than 80%) of the total number of terminal amino groups of linear polylysine.

### [Compound (B)]

The compound of the present invention, wherein the carbonyl group of serine is directly linked by a peptide bond to at least 50% (preferably not less than 60%, more preferably not less than 70%, particularly preferably not less than 80%) of the total number of terminal amino groups of linear polylysine.

The number average molecular weight of the compound of the present invention is not less than 1000 Da, preferably not less than 3000 Da, more preferably not less than 5000 Da. While the number average molecular weight of the compound of the present invention does not have a particularly upper limit, it is desirably not more than 50000 Da, preferably not more than 35000 Da, more preferably not more than 25000 Da because handling is easy. The number average molecular weight measurement can be calculated from the measurement results of proton nuclear magnetic resonance (¹H-NMR), matrix-assisted laser desorption/ionization time-of-flight mass spectrometer (MALDI-TOF-MS) or the like.

The compound of the present invention also encompasses a salt form. Examples of the salt of the compound of the present invention include salts with inorganic acids, salts with organic acids, salts with inorganic bases, salts with organic bases, salts with amino acids and the like.

Examples of the salt with inorganic acid include salts with hydrochloric acid, nitric acid, sulfuric acid, phosphoric acid, hydrobromic acid, hydrofluoric acid, hydroiodic acid, perchloric acid and the like.

Examples of the salt with organic acid include salts with acetic acid, trifluoroacetic acid, trichloroacetic acid, propionic acid, oxalic acid, maleic acid, citric acid, fumaric acid, lactic acid, malic acid, succinic acid, tartaric acid, gluconic acid, ascorbic acid, methanesulfonic acid, trifluoromethanesulfonic acid, benzenesulfonic acid, p-toluenesulfonic acid and the like.

Examples of the salt with inorganic base include salts with sodium salt, potassium salt, calcium salt, magnesium salt, ammonium salt and the like.

Examples of the salt with inorganic base include salts with methylamine, diethylamine, trimethylamine, triethylamine, ethanolamine, diethanolamine, triethanolamine, ethylenediamine, tris(hydroxymethyl)methylamine, dicyclohexylamine, N,N'-dibenzylethylenediamine, guanidine, pyridine, picoline, choline, cinchonine, meglumine and the like.

Examples of the salt with amino acid include salts with lysine, arginine, aspartic acid, glutamic acid and the like.

The compound of the present invention also encompasses a solvate form. The solvate of the compound of the present invention is one in which a molecule of a solvent is coordinated to the compound of the present invention, and includes a hydrate. For example, a hydrate, an ethanol solvate, a dimethyl sulfoxide solvate of the compound of the present invention or a salt thereof can be mentioned.

The compound of the present invention may be labeled with a radiopharmaceutical, a contrast agent or an isotope (e.g., ³H, ²H(D), ¹⁴C, ³⁵S, ⁹⁰Y, ¹¹¹In, ⁶⁷Ga, ⁶⁸Ga, ¹⁷⁷Lu, ⁶⁴Cu, ⁹⁹Tc, ¹⁸⁶Re, ¹⁸⁸Re, ¹³¹I, ¹⁸F etc.). As specific examples, for example, a compound obtained by introducing a chelate group (e.g., diethylenetriamine-N,N,N',N",N"-pentaacetic acid (DTPA) group etc.) into a part of the terminal group of the compound of the present invention to be ¹¹¹In chelate-labeled, a compound obtained by labeling a part of the terminal group of the compound of the present invention with fluorescein isothiocyanate (FITC), a compound obtained by labeling a part of the terminal group of the compound of the present invention with a near infrared probe (e.g., near infrared fluorescent dye, VivoTag 800 (Dye 800)) and the like are also encompassed in the compound of the present invention.

### (Synthesis of the compound of the present invention)

The production method of the compound of the present invention is not particularly limited and, for example, the compound can be synthesized by way of the following reactions.

Poly-L-lysine hydrobromide serving as a starting compound can be easily obtained as commercially available products from Sigma-Aldrich Co. LLC., and the like.

In each of the following steps, the protection or deprotection reaction of a functional group is performed according to a method known per se, for example, Protective Groups in Organic Synthesis, 4th Ed., Theodora W. Greene, Peter G. M. Wuts, Wiley-Interscience (2007) and the like, or a method described in the Examples of the present specification.

The compound obtained in each step in the following reaction formula can be used in the subsequent reaction as a reaction solution or as a crude product. Alternatively, the compound can also be isolated from the reaction mixture according to a conventional method, and can be easily purified by general separation means such as recrystallization, distillation, chromatography and the like.

The compound of the present invention (compound (I)) can be produced, for example, by the following steps.

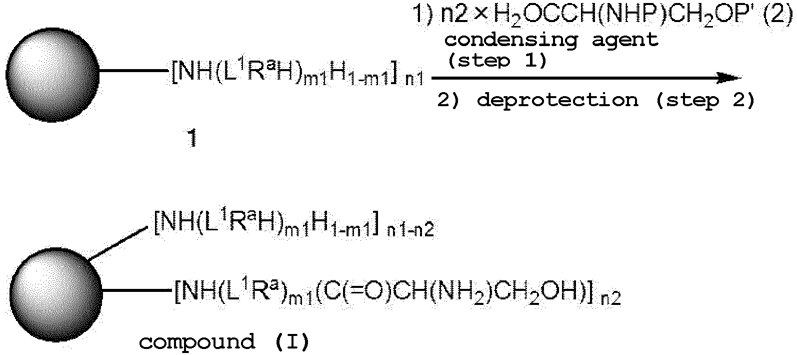

wherein L¹ is a linker (the first linker), R^{a} is an NH group or oxygen atom, P and P' are the same or different and each independently a protecting group, m1 is 0 or 1, n1 is an integer of not less than 8, and n2 is an integer of not less than 4.

### Step 1

In this step, dehydration condensation of the terminal amino group or hydroxy group of linear polylysine (or compound in which a linker (the first linker) is bonded to linear polylysine) (compound 1), and the α-carboxy group of serine having protected amino group and hydroxy group (compound 2) is conducted.

The reaction is carried out in a solvent that does not influence the reaction, and under dehydration condensation reaction conditions known per se.

The amount of compound 2 to be used is generally 0.5 - 3 mol, preferably 1 - 1.5 mol, based on the total number (1 mol) of terminal groups of compound 1.

Examples of the condensing agent to be used for the dehydration condensation reaction include 1-ethyl-3-(3'-dimethylaminopropyl)carbodiimide (WSC), dicyclohexylcarbodiimide (DCC), diisopropylcarbodiimide (DIC), N-ethyl-N'-3-dimethylaminopropylcarbodiimide and hydrochloride thereof (EDC_{•}HCl), (benzotriazol-1-yloxy)tripyrrolidino phosphonium hexafluorophosphate (PyBop), O-(benzotriazol-1-yl)-N,N,N',N'-tetramethyluronium tetrafluoroborate (TBTU), 1-[bis(dimethylamino)methylene]-5-chloro-1H-benzotriazolium 3-oxide hexafluorophosphate (HCTU), O-benzotriazole-N,N,N',N'-tetramethyluronium hexafluoroborate (HBTU) and the like.

In the condensation step, a condensation additive (e.g., 1-hydroxybenzotriazole (HOBt), 1-hydroxy-1H-1,2,3-triazole-5-carboxylic acid ethyl ester (HOCt), 1-hydroxy-7-aza benzotriazole (HOAt) etc.) and a base (e.g., organic base such as triethylamine, pyridine, N,N-diisopropylethylamine and the like, etc.) can be added where necessary.

The amount of the condensing agent to be used is generally 0.5 - 3 mol, preferably 1 - 1.5 mol, based on the total number (1 mol) of terminal groups of compound 1.

Examples of the solvent include aromatic hydrocarbons such as toluene, xylene and the like; amide solvents such as N,N-dimethylformamide, N,N-dimethylacetamide and the like; ethers such as diethyl ether, tetrahydrofuran, dioxane and the like; halogenated hydrocarbons such as chloroform, dichloromethane and the like and a mixture thereof. Among these, N,N-dimethylformamide, dichloromethane and the like are preferable.

The reaction temperature is generally -10 to 30°C, preferably 0°C - 20°C, and the reaction time is generally 1 to 30 hr.

### Step 2

In this step, the protecting groups P and P' of the dehydration condensation product obtained in the aforementioned step 1 are removed to synthesize compound (I).

The reaction is carried out in a solvent that does not influence the reaction, and under deprotection conditions known per se.

The reaction conditions (reaction agent, reaction solvent, reaction temperature, reaction time etc.) of deprotection vary depending on the kind of the protecting groups (P and P'). For example, it can be performed according to the method described in Protective Groups in Organic Synthesis, 4th Ed., Theodora W. Greene, Peter G. M. Wuts, Wiley-Interscience (2007), or the Examples of the present specification, or a method analogous thereto.

### (Medicament of the present invention, and a production method thereof)

A carrier for drug delivery that is composed of the compound of the present invention and selectively delivers a drug to a target tissue (kidney) in a living body is bound to the drug by a covalent bond directly or via a linker to form the medicament (pharmaceutical compound) of the present invention, or encapsulates a drug by noncovalent interaction of a linker introduced into the carrier for drug delivery and the drug to form the medicament (pharmaceutical composition) of the present invention.

As the medicament of the present invention, the following medicaments are preferred.

### [Medicament (A)]

The medicament of the present invention containing a drug directly bound to a part of a serine-derived terminal amino group of a serine-modified moiety of the compound of the present invention (preferably 1 - 40%, more preferably 5 - 20%, based on the total number of serine-derived amino groups).

### [Medicament (B)]

The medicament of the present invention containing a drug directly bound to a part of a terminal amino group of a non-serine-modified linear polylysine of the compound of the present invention (preferably 1 - 40%, more preferably 5 - 20%, based on the total number of terminal amino groups).

### [Medicament (C)]

The medicament of the present invention containing a drug bound via one or more bifunctional linkers (the aforementioned second linkers) to a part of a serine-derived terminal amino group of a serine-modified moiety of the compound of the present invention (preferably 1 - 40%, more preferably 5 - 20%, based on the total number of serine-derived amino groups).

### [Medicament (D)]

The medicament of the present invention containing a drug bound via one or more bifunctional linkers (the aforementioned second linkers) to a part of a terminal amino group of a non-serine-modified linear lysine of the compound of the present invention (preferably 1 - 40%, more preferably 5 - 20%, based on the total number of terminal amino groups or hydroxy groups).

### [Medicament (E)]

The medicament of the present invention containing a drug encapsulated in a molecular recognition moiety by noncovalent interaction resulting from direct binding of a molecular recognition moiety (e.g., inclusion moiety, chelate moiety etc.) to a part of a serine-derived terminal amino group of a serine-modified moiety of the compound of the present invention (preferably 1 - 40%, more preferably 5 - 20%, based on the total number of serine-derived amino groups).

### [Medicament (F)]

The medicament of the present invention containing a drug encapsulated in a molecular recognition moiety by noncovalent interaction resulting from binding of a molecular recognition moiety (e.g., inclusion moiety, chelate moiety etc.) via a linker (e.g., one or more bifunctional linkers (the aforementioned second linkers)) to a part of a serine-derived terminal amino group of a serine-modified moiety of the compound of the present invention (preferably 1 - 40%, more preferably 5 - 20%, based on the total number of serine-derived amino groups).

When the medicament of the present invention is produced by a covalent bond formation of the compound of the present invention and a drug, as described above, it is produced by binding a drug directly or via the aforementioned one or more bifunctional linkers (the second linkers) to a serine-derived terminal amino group of a serine-modified moiety of the compound of the present invention, or directly or via the aforementioned one or more bifunctional linkers (the second linkers) to a terminal amino group of a non-serine-modified linear polylysine. The production method of such medicament is not particularly limited. In a specific embodiment, for example, a method via a general bond-forming reaction, or the like in the field of synthetic organic chemistry such as nucleophilic substitution reaction, electrophilic substitution reaction, dehydration condensation reaction, and the like described in Larock, R. C., Comprehensive Organic Transformations (2nd) Second Edition, Wiley VCH (1999), and the like, the method described in the aforementioned patent document 1 (WO 2019/009436), the method described in the below-mentioned Examples, or a production method analogous thereto, and the like can be mentioned.

When the medicament of the present invention is produced by encapsulating a drug by noncovalent interaction of a linker introduced into a carrier for drug delivery and the drug, as described above, it is produced by binding a molecular recognition moiety (e.g., inclusion moiety, chelate moiety etc.) directly or via the aforementioned one or more bifunctional linkers (the second linkers) to a serine-derived terminal amino group of a serine-modified moiety of the compound of the present invention to cause noncovalent interaction such as hydrophobic interaction, chelate binding and the like between the molecular recognition moiety and the drug, as a result of which the drug is incorporated in the carrier for drug delivery to produce the medicament of the present invention. The production method of such medicament is not particularly limited. In a specific embodiment, for example, in the below-mentioned Example 2 and Experimental Examples, according to a method known per se (e.g., Hnatowich, D. J. et al., Int. J. Appl. Radiat. Isot., 1982, 33(5), 327-332; Nishikawa, M. et al., Biol. Pharm. Bull., 1999, 22(2), 214-218), pharmacokinetics were traced using the medicament of the present invention (¹¹¹In-labeled compound (Ia)) in which ¹¹¹In was chelated to a carrier for drug delivery which was prepared by binding a bifunctional chelating agent, diethylenetriaminepentaacetic acid anhydride (DTPA anhydride), to a part of the terminal amino groups (preferably, one site) of compound (Ia).

The medicament of the present invention shows high renal distribution, can accumulate a drug selectively in the kidney, and has high drug releasability. Thus, it can show superior effects as a prophylactic or therapeutic agent for various renal diseases such as renal insufficiency, inflammation of the kidney and the like. Specific examples of such renal diseases include glomerulonephritis, IgA nephropathy, diabetic nephropathy, membranous nephropathy, hydronephrosis, contrast agent nephropathy, pyelonephritis, renal failure, acute nephritis, chronic nephritis, uremia, interstitial nephritis, kidney disease, nephrotic syndrome, hypertensive nephrosclerosis, diabetic glomerulosclerosis, kidney calculus, amyloid kidney, kidney intravenous thrombosis, Alport syndrome, kidney tumor and the like.

The administration route of the medicament of the present invention is largely divided into oral administration and parenteral administration. The dose of the medicament of the present invention varies depending on the kind of the drug, administration route, administration frequency, age, body weight, pathology and severity of the subject of administration, and the like. It is generally 0.005 - 150 mg/kg/day, preferably, 0.05 - 20 mg/kg/day, and can be administered once or in several portions.

The medicament of the present invention is generally administered in the form of a pharmaceutical composition prepared by mixing with carriers for medicament. Preferable specific examples include external preparations such as microneedle, inhalant, nasal drop and the like, and injections including intravenous injection, intradermal injection, subcutaneous injection, intracavity injections such as intraperitoneal injection and the like. These pharmaceutical compositions are prepared according to conventional methods.

Specific examples the base of a microneedle include polymer bases such as polyvinylpyrrolidone, hyaluronic acid, polyglycolic acid, chondroitin sulfate, carboxymethylcellulose, maltose, dextran, polylactic acid and poly(lactic acid-co-glycolic acid), metal such as stainless steel and the like, silicon, titanium and the like. It can be produced by applying to a surface of the microneedle or placing in the inside thereof.

Inhalant can be produced by powdering or liquidifying the compound of the present invention, blending it into an inhalable spray or carrier, and filling the blend in, for example, an inhalant container such as metered-dose inhaler, dry powder inhaler and the like. It may be a propellant, an aerosol or a spray. As the inhalation propellant, those conventionally known can be widely used, and examples thereof include Freon gas such as Freon-11, Freon-12, Freon-21, Freon-22, Freon-113, Freon-114, Freon-123, Freon-142c, Freon-134a, Freon-227, Freon-C318, 1,1,1,2-tetrafluoroethane and the like, alternative Freon gas such as HFA-227, HFA-134a and the like, hydrocarbon gas such as propane, isobutane, butane and the like, diethyl ether, nitrogen gas, carbon dioxide gas and the like. As the carrier, those conventionally known can be widely used, and examples thereof include saccharides, sugar alcohols, amino acids and the like.

In the case of liquid for inhalation, it is prepared by appropriately selecting preservative (benzalkonium chloride, paraben etc.), colorant, buffering agent (sodium phosphate, sodium acetate etc.), isotonic agent (sodium chloride, concentrated glycerol etc.), thickener (carboxyvinyl polymer etc.), preservative (benzalkonium chloride, paraben etc.), absorption promoter and the like as necessary.

In the case of powder for inhalation, it is prepared by appropriately selecting lubricant (stearic acid and a salt thereof etc.), binder (starch, dextrin etc.), excipient (lactose, cellulose etc.), colorant, absorption promoter and the like as necessary.

Nasal drop can take various forms such as a dropping type, an application type, a spray type and the like. In the case of the spray type, a manual pump type nasal drop with a mechanism to eject the liquid by manually moving the pump attached to the container, an aerosol-type nasal drop having a mechanism to automatically eject a liquid agent by filling the container with a propellant agent and moving a valve attached to the container and the like are also included.

Injection is prepared by dissolving the medicament of the present invention in distilled water for injection, and further, a solution containing a solubilizing agent, a buffer, a pH adjuster, an isotonic agent, a soothing agent, a preservative, and the like, where necessary, or suspending the compound in distilled water for injection or vegetable oil. In this case, a base, a suspending agent, a thickener and the like can be added as necessary. In addition, it may be in a form for dissolving a powder or a freeze-dried product when in use, and an excipient (e.g., mannitol, sorbitol, lactic acid, trehalose, sucrose etc.) and the like can be added as necessary.

The content of the medicament of the present invention in a pharmaceutical composition varies depending on the dosage form thereof. It is generally 0.0025 - 50 wt% of the whole composition. Such pharmaceutical composition may also contain other drugs effective for treatment (other drugs, etc. for preventing or treating renal diseases). In combined use, the administration period of the compound of the present invention and a concomitant drug is not particularly limited, and the mixing ratio of the medicament of the present invention and the concomitant drug can be appropriately determined according to the subject of administration, administration method, disease, combination and the like. For example, while the content of a concomitant drug in the combination drug of the present invention varies depending on the form of the preparation, it is generally 0.0025 - 50 wt% of the whole composition.

While the present invention is described in more detail in the following preferred Examples and Experimental Examples of the present invention, the following Examples are only for exemplary confirmation of the effects of the present invention and do not limit the present invention to them. In addition, the present invention may be modified without departing from the scope of the invention.

### [Example]

The present invention is explained in more detail in the following by referring to Examples, Formulation Example and Experimental Examples, which do not limit the present invention. In addition, the present invention may be modified without departing from the scope of the invention.

Unless otherwise specified, the apparatus, reagents, and the like used in the Examples can be easily obtained or prepared according to a method generally employed in the art, or are commercially available.

The "room temperature" in the following Examples is generally about 10°C to about 25°C.

### Example 1: Synthesis of kidney targeting carrier for drug delivery (serine-modified poly-L-lysine) (compound of the present invention (compound (Ia))

1.1 equivalents of Boc-Ser(t-Bu)-OH (manufactured by Watanabe Chemical Industries, Ltd.), 1.1 equivalents of 1-[bis(dimethylamino)methylene]-1H-benzotriazolium 3-oxide hexafluorophosphate (HBTU) (manufactured by Merck Millipore), 1.1 equivalents of anhydrous 1-hydroxy-1H-benzotriazole (HOBt) (manufactured by Watanabe Chemical Industries, Ltd.) and 2.2 equivalents of N,N-diisopropyl ethylamine (DIPEA), each to the total number of amino groups of poly-L-lysine hydrobromide (manufactured by Sigma-Aldrich Co. LLC., P6516-100M, number average molecular weight: 4000 - 15000) were mixed in DMF/DMSO (1:1). Then, the reaction mixture was reacted by stirring at room temperature until the ninhydrin test yielded negative results on TLC analysis. After completion of coupling, this solution was purified by precipitation with diethylether three times. The precipitates thereof were dissolved in a trifluoroacetic acid (TFA) cocktail (95% TFA, 2.5% thioanisole (TIS) and 2.5% purified water) to deprotect the Boc group and t-Bu group (protecting group of oxygen atom of serine). Then, the reaction mixture was incubated at room temperature for 90 min. After completion of deprotection, the reaction mixture was purified by precipitation with diethylether three times. The crude precipitates were dissolved in ultrapure water and passed through PD-10 column to separate the resulting products by size-exclusion chromatography, and then lyophilized to obtain serine-modified poly-L-lysine (hereinafter to be referred to as "compound (Ia)"). For the analysis of particles size and zeta potential, compound (Ia) was dissolved in phosphate buffered saline (PBS) (pH 7.4) at a concentration of 1 mg/mL. Then, these properties were measured by Zetasizer Nano ZS (Malvern Instruments, Worcestershire, UK) for the mean particle size and zeta potential of compound (Ia).

Table 1 shows physicochemical property of compound (Ia) and poly-L-lysine (the aforementioned poly-L-lysine hydrobromide).

**[Table 1]**

| | mean particle size (nm) | zeta potential (mV) |
|---|---|---|
| poly-L-lysine | 4.25±0.62 | 13.1±1.56 |
| compound (Ia) | 6.02±0.34 | 10.4±0.15 |

According to Table 1, the particle size of the obtained compound (Ia) was about 6.0 nm (6.02±0.34 nm) and zeta potential was about 10.4 mV (10.4±0.15 mV), whereas the particle size of poly-L-lysine was about 4.3 nm (4.25±0.62 nm) and the zeta potential was about 13.1 mV (13.1±1.56 mV).

### Control Example: Synthesis of ¹¹¹In-labeled poly-L-lysine (compound of Control Example)

According to a method known per se (Hnatowich, D. J. et al., Int. J. Appl. Radiat. Isot., 1982, 33(5), 327-332; Nishikawa, M. et al., Biol. Pharm. Bull., 1999, 22(2), 214-218), a bifunctional chelating agent, diethylenetriaminepentaacetic acid anhydride (DTPA anhydride), was bonded to poly-L-lysine hydrobromide, and then radioisotope-labeled with ¹¹¹In to synthesize ¹¹¹In-labeled poly-L-lysine. Specifically, poly-L-lysine was dissolved in 0.1M HEPES buffer (pH 7, 1 mL), 10 µL (2 equivalents of poly-L-lysine) of diethylenetriamine-N,N,N',N",N"-pentaacetic acid (DTPA) anhydride (manufactured by DOJINDO LABORATORIES) dissolved in DMSO was added, and the mixture was reacted for 30 min at room temperature. Unreacted DTPA anhydride was removed by gel filtration using PD10 column (manufactured by GE Healthcare), and the solvent was substituted with 0.1M citrate buffer (pH 5.5) by ultrafiltration and concentrated. Then, 40 µL of DTPA-bonded poly-L-lysine dissolved in 0.1M citrate buffer (pH 5.5) and 20 µL of [¹¹¹In] InCl₃ solution (74 MBq/mL) (manufactured by Nihon Medi-Physics Co., Ltd.) were mixed, and labeling was performed by allowing the mixture to stand at room temperature for 20 min. The mixture was purified by gel filtration and ultrafiltration using PD10 column.

The physicochemical properties (particle size and zeta potential) of ¹¹¹In-labeled poly-L-lysine are the same as those of poly-L-lysine.

### Example 2: Synthesis of ¹¹¹In-labeled serine-modified poly-L-lysine (¹¹¹In-labeled compound (Ia))

According to a method known per se (Hnatowich, D. J. et al., Int. J. Appl. Radiat. Isot., 1982, 33(5), 327-332; Nishikawa, M. et al., Biol. Pharm. Bull., 1999, 22(2), 214-218), a bifunctional chelating agent, diethylenetriaminepentaacetic acid anhydride (DTPA anhydride), was bonded to compound (Ia), and then radioisotope-labeled with ¹¹¹In to synthesize the medicament of the present invention (¹¹¹In-labeled compound (Ia)). Specifically, compound (Ia) was dissolved in 0.1M HEPES buffer (pH 7, 1 mL), 10 µL (2 equivalents of compound (Ia)) of diethylenetriamine-N,N,N',N",N"-pentaacetic acid (DTPA) anhydride (manufactured by DOJINDO LABORATORIES) dissolved in DMSO was added, and the mixture was reacted for 30 min at room temperature. Unreacted DTPA anhydride was removed by gel filtration using PD10 column (manufactured by GE Healthcare), and the solvent was substituted with 0.1M citrate buffer (pH 5.5) by ultrafiltration and concentrated. Then, 40 µL of DTPA-bonded compound (Ia) dissolved in 0.1M citrate buffer (pH 5.5) and 20 - 300 µL of [¹¹¹In] InCl₃ solution (74 MBq/mL) (manufactured by Nihon Medi-Physics Co., Ltd.) were mixed, and labeling was performed by allowing the mixture to stand at room temperature for 20 min. The mixture was purified by gel filtration and ultrafiltration using PD10 column.

The physicochemical properties (particle size and zeta potential) of ¹¹¹In-labeled compound (Ia) are the same as those of compound (Ia).

### Example 3: Synthesis of FITC-labeled serine-modified poly-L-lysine (FITC-labeled compound (Ia))

Compound (Ia) was dissolved in 0.1M HEPES buffer (pH 7, 1 mL), 10 µL (2 equivalents of compound (Ia)) of fluorescein isothiocyanate isomer (manufactured by Sigma-Aldrich Co. LLC.) was added, and the mixture was reacted for 30 min at room temperature. Unreacted fluorescein isothiocyanate isomer was removed by gel filtration using PD10 column (manufactured by GE Healthcare), and the residue was purified by ultrafiltration.

The physicochemical properties (particle size and zeta potential) of FITC-labeled compound (Ia) are the same as those of compound (Ia).

### Comparative Example 1: ¹¹¹In-labeled serine-modified polyamidoamine dendrimer (¹¹¹In-labeled serine-modified PAMAM(G3))_

¹¹¹In-labeled serine-modified PAMAM(G3) was synthesized by a method similar to that of Example 2 except that serine-modified PAMAM(G3) synthesized according to the method described in Example 1 of WO 2019/009436 was used as a starting compound instead of compound (Ia) in the above-mentioned Example 2.

The particle size of serine-modified PAMAM(G3) was about 4 nm (4.03±0.09 nm) and the zeta potential was about 4.8 mV (4.76±0.70 mV). Therefore, it can be said that the physicochemical properties (particle size and zeta potential) of ¹¹¹In-labeled serine-modified PAMAM(G3) were also the same.

### Comparative Example 2: ¹¹¹In-labeled polyamidoamine dendrimer (¹¹¹ In-labeled PAMAM(G3))

¹¹¹In-labeled PAMAM(G3) was synthesized by a method similar to that of Example 2 except that a third generation polyamidoamine dendrimer (PAMAM(G3)) (manufactured by Sigma-Aldrich Co. LLC.) was used as a starting compound instead of compound (Ia) in the above-mentioned Example 2.

The particle size of the compound of Comparative Example 2 was about 4.2 nm (4.20±0.09 nm) and the zeta potential was about 4.6 mV (4.56±0.81 mV). Therefore, it can be said that the physicochemical properties (particle size and zeta potential) of ¹¹¹In-labeled PAMAM(G3) were also the same.

### Experimental Example 1: Evaluation of pharmacokinetics of compound (Ia)

### (Test method)

¹¹¹In-labeled compound (Ia) was intravenously administered to ddY mouse, and pharmacokinetics of compound (Ia) were evaluated. To be specific, ¹¹¹In-labeled compound (Ia) obtained in the above-mentioned Example 2 was intravenously administered to ddY mouse via the tail vein. At a suitable time point after intravenous injection, the blood was collected from the abdominal vena cava under isoflurane anesthesia. Liver, kidney, spleen, heart and lung tissue were excised, rinsed with saline, blotted dry, and the wet weight of the organs was measured.

The collected blood was centrifuged at 2000×g for 5 min to obtain plasma. The samples of the collected organs and 100 µL of plasma were transferred to counter tubes, and the radioactivity of each sample was measured using a gamma counter (1480 WizardTM 3', Perkin-Elmer, Boston, MA, USA). In addition, the pharmacokinetics of poly-L-lysine were also evaluated by performing similar experiments using ¹¹¹In-labeled poly-L-lysine of Control Example.

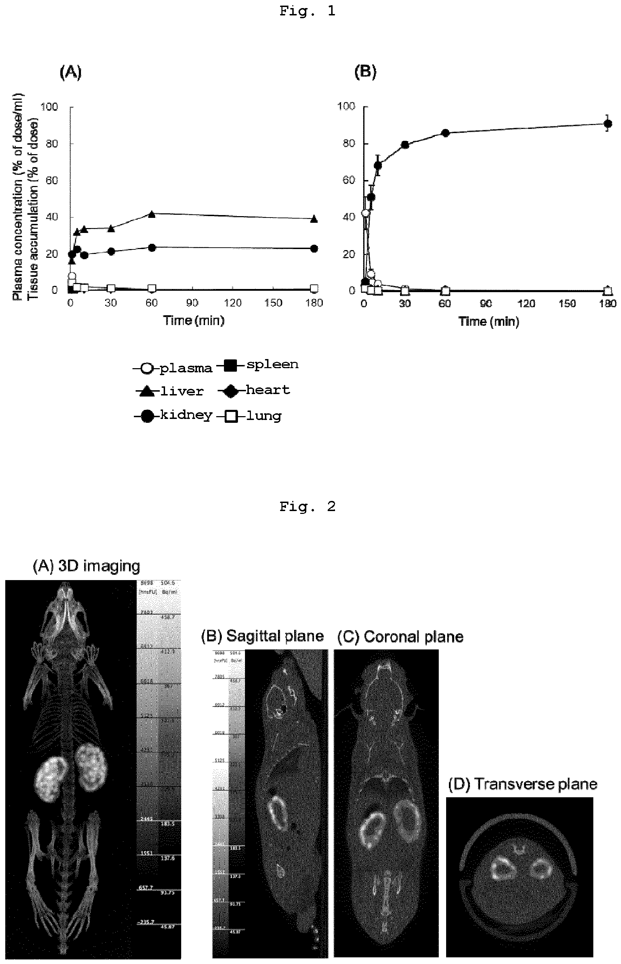

Fig. 1 shows the concentration-time profiles of plasma, liver, kidney, spleen, heart and lung after intravenous injection of ¹¹¹In-labeled poly-L-lysine of Control Example (Fig. 1(A)) and ¹¹¹In-labeled compound (Ia) (Fig. 1(B)) (i.e., pharmacokinetics of poly-L-lysine and compound (Ia)).

According to Fig. 1(B), about 90% of the dose of compound (Ia) at 3 hr after intravenous administration was accumulated in kidney. Accumulation in other organs was scarcely found, and the compound showed pharmacokinetics superior in renal selectivity. In contrast, non-serine-modified poly-L-lysine (Fig. 1(A)) showed low renal distribution as compared with compound (Ia), also showed distribution in the liver, and low kidney selectivity.

From the results of Fig. 1, it was confirmed that compound (Ia) is particularly superior in renal distribution and renal selectivity.

### Experimental Example 2: Observation of organ distribution of compound (Ia) by single photon emission computed tomography/computed tomography (SPECT/CT) imaging

### (Test method)

SPECT/CT was performed using g-CUBE(SPECT)/X-CUBE(CT) (Molecubes, Gent, Belgium). ¹¹¹In-labeled compound (Ia) (15.6 MBq/mouse) was injected intravenously into the mouse. At 3 hr after intravenous administration of ¹¹¹In-labeled compound (Ia), under isoflurane anesthesia, a 1 hr SPECT scan of the mouse was obtained. Prior to the SPECT scan, under isoflurane inhalation anesthesia, a CT scan of the mouse was performed for anatomical reference. The SPECT image was reconstructed, and image analysis was performed by using VivoQuant (version 5.1; inviCRO, Hillsboro, OR, USA).

### (Results)

The results are shown in Fig. 2. Fig. 2 shows SPECT/CT images ((A) 3D images, (B) sagittal plane image, (C) coronal plane image, and (D) transverse plane image) of organ distribution after intravenous injection of ¹¹¹In-labeled compound (Ia). According to Fig. 2, ¹¹¹In-labeled compound (Ia) was specifically accumulated in the kidney. In the kidney, ¹¹¹In-labeled compound (Ia) was mainly accumulated in the cortex part where proximal tubules and the like are present. Since proximal tubule is the site of onset of renal diseases such as renal cell carcinoma, chronic renal failure and the like, these study results suggest that ¹¹¹In-labeled compound (Ia) may be applicable to image diagnosis of renal diseases.

### Experimental Example 3: Distribution of compound (Ia) in kidney

### (Test method)

FITC-labeled compound (Ia) obtained in Example 3 was administered to ddY mouse from the tail vein. At 60 min after intravenous administration, the kidneys were excised under isoflurane anesthesia, fixed with OCT, and frozen. Frozen kidney sections were treated with 100 µg/mL 4',6-diamidino-2-phenylindole (DAPI) (manufactured by Wako Pure Chemical Industries, Ltd.), and stained kidney sections were observed under a fluorescence microscope (Biozero, KEYENCE, Osaka, Japan).

### (Results)

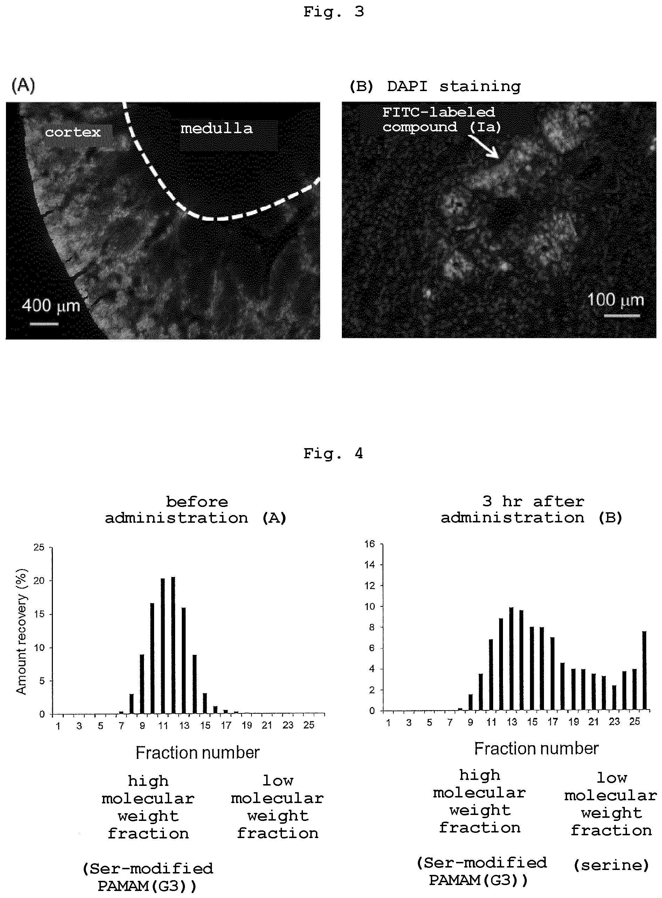

The results are shown in Fig. 3. Fig. 3 (A) and (B) show microscopic images of kidney sections of a mouse 60 min after intravenous administration of FITC-labeled compound (Ia). According to Fig. 3(A), it was confirmed that the fluorescence of FITC-labeled compound (Ia) was remarkably high in the renal cortex of renal medulla. An enlarged microscopic image of renal cortex is shown in Fig. 3(B). Strong fluorescence derived from FITC-labeled compound (Ia) was observed in the proximal tubules. From the above, it was confirmed that compound (Ia) is mainly distributed in the proximal tubules of kidney. Since proximal tubule is the site of onset of renal diseases such as kidney cancer, chronic renal failure and the like, compound (Ia) was found to show distribution in kidney which is advantageous for the treatment of renal diseases and image diagnosis.

### Experimental Example 4: Evaluation of releasability of serine from compound (Ia) and biodegradability of compound (Ia)

### (Test method)

The ¹¹¹In-labeled serine-modified PAMAM(G3) obtained in Comparative Example 1, ¹¹¹In-labeled compound (Ia) obtained in Example 2, ¹¹¹In-labeled PAMAM(G3) obtained in Comparative Example 2, and ¹¹¹In-labeled poly-L-lysine which is the compound of Control Example were each intravenously administered and, after sacrifice by abdominal vena cava amputation 3 hr after administration, the residual blood in the kidney was removed by passing physiological saline from the left ventricle. The kidney after blood removal was immediately ice-cooled, purified water (4 mL) was added and homogenized. A saturated potassium chloride solution (1 mL) was added to the prepared kidney homogenate, and the mixture was allowed to stand at 4°C overnight and then centrifuged. The centrifuged supernatant (1 mL) was developed on a PD-10 column with 0.1 M acetate buffer (pH 6.0), and the radioactivity of the fraction was measured.

### (Results)

The results are shown in Fig. 4 - Fig. 7.

According to Fig. 4, ¹¹¹In-labeled serine-modified PAMAM(G3) showed radioactivity on the high molecular weight fraction side in the samples before administration (Fig. 4 (A)), and showed radioactivity mainly on the high molecular weight fraction side, relatively low molecular weight fraction side, 3 hr after administration in the kidney homogenate samples (Fig. 4 (B)).

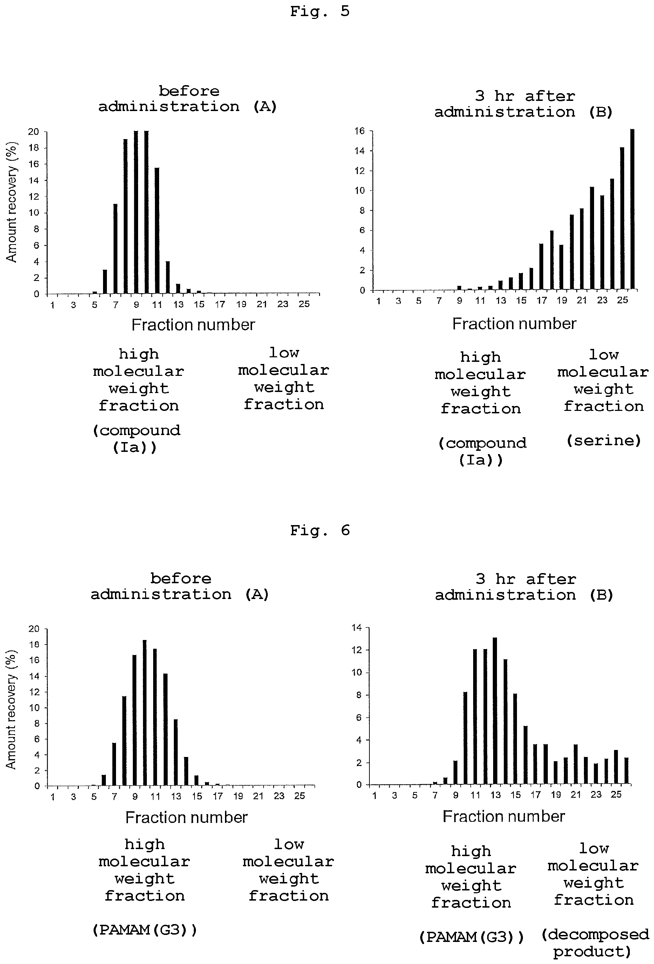

According to Fig. 5, ¹¹¹In-labeled compound (Ia) showed radioactivity on the high molecular weight fraction side in the samples before administration (Fig. 5 (A)), and showed radioactivity mainly on the low molecular weight fraction side 3 hr after administration in the kidney homogenate samples (Fig. 5 (B)).

According to Fig. 6, ¹¹¹In-labeled PAMAM(G3) showed radioactivity on the high molecular weight fraction side in the samples before administration (Fig. 6 (A)), and similarly showed radioactivity mainly on the high molecular weight fraction side 3 hr after administration in the kidney homogenate samples (Fig. 6 (B)).

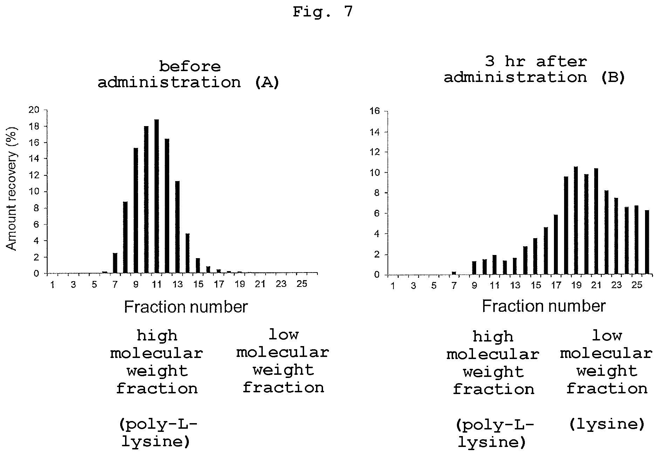

According to Fig. 7, ¹¹¹In-labeled poly-L-lysine showed radioactivity on the high molecular weight fraction side in the samples before administration (Fig. 7 (A)), and showed radioactivity mainly on the low molecular weight fraction side 3 hr after administration in the kidney homogenate samples (Fig. 7 (B)).

From the above results, it was shown that ¹¹¹In-labeled serine-modified PAMAM(G3) released almost no serine in the kidney 3 hr after intravenous administration (Fig. 4 (A)), but ¹¹¹In-labeled compound (Ia) rapidly released serine in the kidney after intravenous administration (Fig. 5 (B)). It was also confirmed that ¹¹¹In-labeled PAMAM(G3) is hardly decomposed in the kidney (Fig. 6 (A) and (B)), and ¹¹¹In-labeled poly-L-lysine (that is, linear polylysine) is easily decomposed in the kidney (Fig. 7 (A) and (B)).

The serine releasability and high decomposability in vivo of the carrier for drug delivery are directly linked to the bioavailability and safety of the drug bound to the drug delivery carrier. Therefore, it can be said that the compound of the present invention (Ia) has particular superiority as a medicament in the bioavailability and safety as compared with serine-modified PAMAM(G3) that has been reported to show extremely high renal distribution.

### Experimental Example 5: Evaluation of acute kidney toxicity after administration of compound (Ia)

### (Test method)

Compound (Ia) (1 mg/kg/day) was intravenously administered to ddY mouse continuously for 5 days, and acute kidney toxicity after intravenous administration was evaluated. The severity of renal damage was evaluated from the plasma creatinine level and plasma urea nitrogen concentration 6 days after the start of administration. The plasma creatinine concentration was measured using a commercially available measurement kit (LabAssay, manufactured by Wako Pure Chemical Industries, Ltd.). Plasma urea nitrogen concentration was measured using a commercially available measurement kit (DIUR-100, manufactured by BioAssySystem). Mercury chloride was subcutaneously injected at 8 mg/kg, the group bred for 2 days was used as a positive control, and the results were compared.

### (Results)

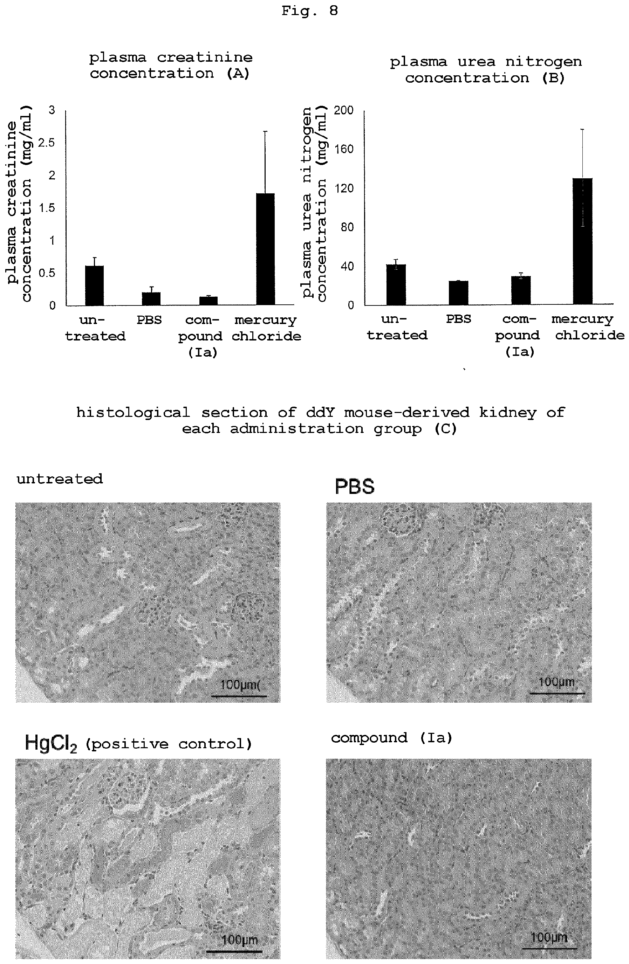

The results are shown in Fig. 8. According to Fig. 8, subcutaneous injection of positive control mercury chloride showed an increase in the plasma creatinine level (Fig. 8 (A)) and urea nitrogen concentration (Fig. 8 (B)), which caused acute nephrotoxicity. However, no increase in the plasma creatinine level (Fig. 8(A)) or plasma urea nitrogen concentration (Fig. 8 (B)) was observed by daily administration of compound (Ia). Furthermore, infiltration and necrosis of inflammatory cells and damaged cells were observed in histological sections of kidney tissue derived from ddY mouse administered with mercury chloride (Fig. 8 (C)). However, the histological section of the kidney tissue after administration of compound (Ia) was almost the same as that of the untreated group or the mouse administered with PBS (Fig. 8 (C)).

### Experimental Example 6: Evaluation of renal cell carcinoma proliferation suppressive activity by ⁹⁰Y-labeled compound (Ia)

### (Test method)

⁹⁰Y-labeled compound (Ia) (hereinafter to be also referred to as "⁹⁰Y-labeled compound (Ia)") known as a radioactive nuclide for the treatment of cancer was intravenously administered to a renal cell carcinoma model mouse, and the renal cell carcinoma proliferation suppressive effect of the ⁹⁰Y-labeled compound (Ia) was evaluated. Specifically, compound (Ia) was labeled with ⁹⁰Y by using diethylenetriamine-N,N,N',N'',N''-pentaacetic acid (DTPA) anhydride (manufactured by DOJINDO LABORATORIES), which is a bifunctional chelating agent, according to the method of Aung et al.(Oncotarget, 7, 38835-38844 (2016)).

The renal cell carcinoma model was produced by injecting firefly luciferase gene-labeled B16-BL6 cells (B16-BL6/Luc cells, 2.5×10⁵ cells) together with Matigel (registered trade mark) Matrix in the renal cortex of the right kidney of C57BL/6J mouse. Immediately after administration of the tumor inoculation, ⁹⁰Y-labeled compound (Ia) was injected into the tail vein at a dose of 1.0 Mq. 14 days after tumor inoculation, 2.5 mg of D-Luciferin was intraperitoneally injected into the mouse under anesthesia by intraperitoneal injection of triple anesthesia (medetomidine (0.3 mg/kg), midazolam (2 mg/kg), butorphanol (2.5 mg/kg)), and imaged using LAS-4000 IVIS Imaging System (FUJIFILM, Japan). Then, the mouse was euthanized, the kidneys were isolated, and the luciferase activity in the tissue was measured with a luminometer (Lumat LB9507, EG&G Berthold, Bad Wild-bad, Germany). From the obtained luciferase activity, the number of cancer cells in the kidney was calculated using a regression line. In addition, the body weight of the mouse was measured immediately after the tumor inoculation administration and 14 days after the tumor inoculation.

### (Results)

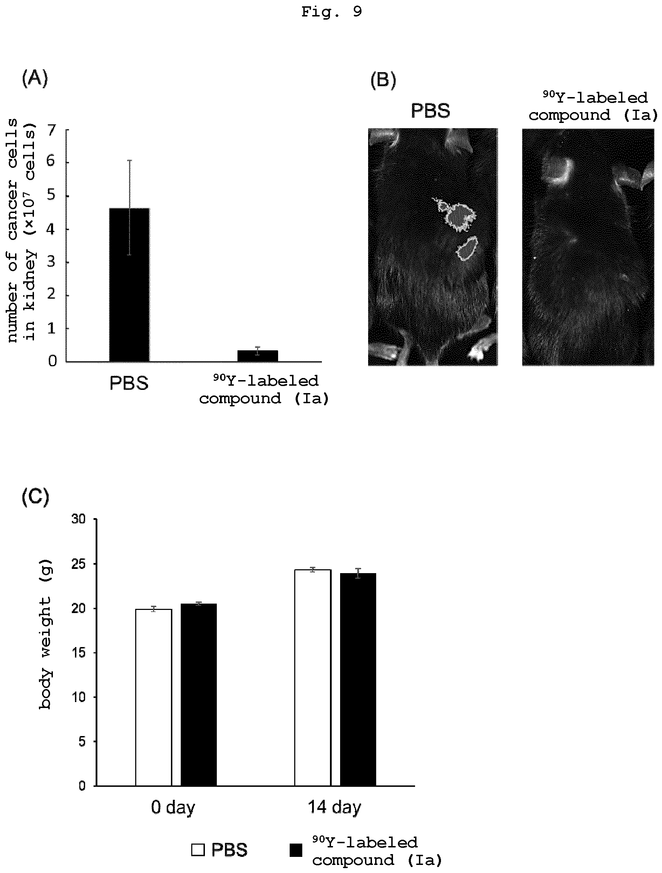

The results are shown in Fig. 9. According to Fig. 9 (A), the administration of ⁹⁰Y-labeled compound (Ia) remarkably suppressed the growth of the number of cancer cells in the kidney. In addition, the luciferase luminescence derived from cancer cells after administration of ⁹⁰Y-labeled compound (Ia) was remarkably suppressed as compared with that of mouse administered with PBS (Fig. 9 (B)). Furthermore, administration of ⁹⁰Y-labeled compound (Ia) did not affect the body weight of mouse (Fig. 9 (C)).

From the above results, it is considered that the proliferation of cancer cells in the kidney was suppressed by the radiation of ⁹⁰Y delivered to the kidney.

Formulation Example (production of freeze-dried preparation (reagent for clinical test))

| | |
|---|---|
| 1) Medicament of the present invention | |
| (¹¹¹In-labeled compound (Ia)) | 40 mg |
| 2) Mannitol | 10 mg |
| 3) Ultrapure water | 1 ml |
| Total | 50 mg/ml |

1), 2) and 3) were mixed, sterilized by filtration with a membrane filter, filled in a container and lyophilized to give a freeze-dried preparation.

### [Industrial Applicability]

The compound of the present invention can be easily synthesized using a commercially available linear polylysine as it is as a starting material. In addition, the compound of the present invention shows high renal selectivity with a particularly high renal distribution rate and scarce distribution to other organs, as well as low toxicity, and is extremely useful as a carrier for drug delivery of prophylactic agents, diagnostic agents (clinical test drugs), therapeutic agents, and the like for various renal diseases. Furthermore, the compound of the present invention and a medicament in which a drug is bound to or encapsulated in the compound is superior in the biodegradability in the kidney and releasability of the serine moiety that binds to the drug. Therefore, they are expected to be applicable to a wide range of use as a low toxic, practical carrier for drug delivery, and a safe and effective medicament.

This application is based on a patent application No. 2019-123540 filed on July 2, 2019 in Japan, the contents of which are incorporated in full herein.

## Claims

1. A compound in which a carbonyl group of serine is linked by a peptide bond or an ester bond directly or via a linker to at least 50% of the total number of terminal amino groups of linear polylysine.

2. The compound according to claim 1, wherein the carbonyl group of serine is directly linked to the terminal amino group of linear polylysine by a peptide bond.

3. The compound according to claim 1 or 2, wherein the linear polylysine has a number average molecular weight of 4000 - 15000Da.

4. A carrier for drug delivery that is composed of the compound according to any one of claims 1 to 3, and selectively delivers a drug to a target tissue in vivo.

5. The carrier for drug delivery according to claim 4, wherein the aforementioned target tissue is kidney.

6. A medicament comprising the carrier for drug delivery according to claim 4 or 5, and a drug bound to the carrier directly or via a linker or encapsulated therein.

7. The medicament according to claim 6, wherein the aforementioned drug is at least one selected from the group consisting of an angiotensin converting enzyme inhibitor, an anti-cancer agent, an anti-inflammatory agent, an anti-infective agent, an anti-fibrotic agent, an immunosuppressant, an antioxidant, a nucleic acid drug, a radiopharmaceutical and a contrast agent.

8. The medicament of any according to claim 6 or 7, wherein the medicament is a diagnostic, prophylactic or therapeutic agent for renal diseases.

9. The medicament according to claim 8, wherein the renal disease is at least one member selected from the group consisting of glomerulonephritis, IgA nephropathy, diabetic nephropathy, membranous nephropathy, hydronephrosis, contrast nephropathy, pyelonephritis, renal failure, acute nephritis, chronic nephritis, interstitial nephritis, renopathy, nephrotic syndrome, hypertensive nephrosclerosis, diabetic glomerulosclerosis, kidney calculus, amyloid nephropathy, renal vein thrombosis, Alport syndrome, and renal tumor.
